# EUROPEAN PATENT APPLICATION

(11) **EP 3 415 183 A2**
(43) Date of publication of application: **19.12.2018**
(21) Application number: 18177683.2
(22) Date of filing: 14.06.2018
(51) Int. Cl.: A61M 1/16, B01J 49/60, B01J 20/34, B01J 49/57, B01J 39/12, B01J 41/10

(54) **ZIRCONIUM OXIDE DISINFECTION RECHARGING AND CONDITIONING**

(30) Priority: 15.06.2017 US 201762520537 P; 20.04.2018 US 201815958396
(71) Applicant: Medtronic, Inc., Minneapolis, Minnesota 55432 (US)
(72) Inventor: PUDIL, Bryant, Plymouth, MN Minnesota 55447 (US); HOBOT, Christopher M., Rogers, MN Minnesota 55374 (US)
(74) Representative: Maschio, Antonio

(57) **Abstract**

The invention relates to systems and methods for disinfecting, recharging, and conditioning zirconium oxide in a reusable sorbent module. The systems and methods provide for reuse by recharging and conditioning a zirconium oxide sorbent module after being used in dialysis.

## Description

### CROSS-REFERENCE TO RELATED APPLICATIONS

This application claims the benefit of and priority to U.S. Provisional Patent Application No. 62/520,537 filed June 15, 2017, the entire disclosure of which is incorporated by reference herein.

### FIELD OF THE INVENTION

The invention relates to systems and methods for disinfecting, recharging, and conditioning zirconium oxide in a reusable sorbent module. The systems and methods provide for reuse by recharging and conditioning a zirconium oxide sorbent module after being used in dialysis.

### BACKGROUND

Zirconium oxide containing sorbent cartridges are used in dialysis to remove phosphate and other anions from dialysate, allowing the dialysate to be reused. Known systems for reusing zirconium oxide require the zirconium oxide to be removed from a sorbent cartridge, transported to a reprocessor, treated, and placed into a new sorbent cartridge, increasing costs and waste associated with reuse of zirconium oxide. Known systems and methods also cannot reuse a zirconium oxide sorbent module by concurrently disinfecting the ZO to reduce the microbial load to acceptable levels, recharging to recover the capacity of the zirconium oxide, and conditioning the zirconium oxide to return the zirconium oxide to an appropriate chemical state for use in dialysis. Hence, there is a need for systems and methods that can quickly, efficiently, and effectively disinfect, recharge and condition zirconium oxide in a reusable sorbent module. There is further a need for systems and methods that are optimized to carry out all three steps with the least amount of waste at reduced cost than known systems and methods.

### SUMMARY OF THE INVENTION

The first aspect of the invention relates to a method for recharging zirconium oxide in a zirconium oxide sorbent module. In any embodiment, the method can include flowing a disinfectant solution through the zirconium oxide sorbent module and flowing a base solution through the zirconium oxide sorbent module; wherein the step of flowing the disinfectant solution through the zirconium oxide sorbent module includes any one of: (a) flowing an acid solution and a peracetic acid solution through the zirconium oxide sorbent module; (b) flowing a heated base solution through the zirconium oxide sorbent module; or (c) flowing a bleach solution through the zirconium oxide sorbent module.

In any embodiment, the base solution can be a sodium hydroxide solution.

In any embodiment, the step of flowing the acid solution and the peracetic acid solution through the zirconium oxide sorbent module can include flowing a mixture of acetic acid and peracetic acid through the zirconium oxide sorbent module. In any embodiment, the peracetic acid solution can also contain hydrogen peroxide.

In any embodiment, the mixture of acetic acid and peracetic acid can contain between 1% and 3% peracetic acid and between 5% and 15% acetic acid. In any embodiment, the peracetic acid solution can also contain hydrogen peroxide.

In any embodiment, the step of flowing the acid solution and the peracetic acid solution through the zirconium oxide sorbent module can include first flowing the acid solution through the zirconium oxide sorbent module; and then flowing the peracetic acid solution through the zirconium oxide sorbent module.

In any embodiment, the acid solution can contain acetic acid and sodium acetate.

In any embodiment, the acid solution can contain between 0.3M and 1.1M sodium acetate and between 0.2M and 0.8M acetic acid.

In any embodiment, the acid solution can further contain between 2.5 M and 4.9 M sodium chloride.

In any embodiment, the steps of flowing the base solution and flowing the disinfectant solution through the zirconium oxide sorbent module can include flowing a mixture of the base solution and disinfectant solution through the zirconium oxide sorbent module.

In any embodiment, the mixture of the base solution and disinfectant solution can be a mixture of sodium hydroxide and bleach.

In any embodiment, the mixture of sodium hydroxide and bleach can contain between 0.01% and 2.0 % bleach and between 0.2 M and 2.0 M sodium hydroxide.

In any embodiment, a flow rate of the mixture of the base solution and disinfectant solution can be between 5 and 500 mL/min.

In any embodiment, the volume of the mixture of the base solution and disinfection solution flowed through the zirconium oxide sorbent module can be between 1.0 and 3.0 L.

In any embodiment, the method can include rinsing the zirconium oxide sorbent module with water after flowing the base solution through the zirconium oxide sorbent module.

In any embodiment, a volume of water flowed through the zirconium oxide sorbent module to rinse the zirconium oxide sorbent module can be between 100% and 1000% of a void volume of the zirconium oxide sorbent module.

In any embodiment, the method can include conditioning the zirconium oxide sorbent module by flowing a sodium bicarbonate solution through the zirconium oxide sorbent module.

In any embodiment, a pH of the sodium bicarbonate solution can be between 5.0 and 9.0.

In any embodiment, the method can include generating either or both of the disinfectant solution and base solution in a zirconium oxide recharging flow path.

In any embodiment, the method can include the step of flowing the sodium bicarbonate solution through a zirconium phosphate sorbent module prior to flowing the sodium bicarbonate solution through the zirconium oxide sorbent module.

In any embodiment, any one of (a) the step of flowing the disinfectant solution through the zirconium oxide sorbent module can include flowing a concentrated disinfectant solution and water to a static mixer to generate a dilute disinfectant solution; and flowing the dilute disinfectant solution through the zirconium oxide sorbent module; (b) the step of flowing the base solution through the zirconium oxide sorbent module can include flowing a concentrated base solution and water to a static mixer to generate a dilute base solution; and flowing the dilute base solution through the zirconium oxide sorbent module; or (c) combinations thereof.

The features disclosed as being part of the first aspect of the invention can be in the first aspect of the invention, either alone or in combination.

The second aspect of the invention is drawn to a zirconium oxide recharging flow path. In any embodiment, the zirconium oxide recharging flow path can include (i) a water source, a disinfectant source, a base source, and an acetic acid source; (ii) a zirconium oxide module inlet and a zirconium oxide module outlet; wherein the zirconium oxide module inlet and the zirconium oxide module outlet are fluidly connectable to a zirconium oxide sorbent module; (iii) at least one pump for flowing fluid from the water source, the disinfectant source, the base source, and the acetic acid source to the zirconium oxide module inlet; and (iv) a zirconium oxide effluent line fluidly connectable to the zirconium oxide module outlet.

In any embodiment, the disinfectant source can be a peracetic acid source.

In any embodiment, the acetic acid source can contain a mixture of acetic acid, sodium chloride, and sodium acetate.

In any embodiment, the disinfectant source can be a peracetic acid solution having a concentration in a range between 0.5 % and 2% of peracetic acid in water.

In any embodiment, the acetic acid source can contain sodium chloride between 2.5 M and 4.9 M, sodium acetate between 0.3 M and 1.1 M, and acetic acid between 0.2 M and 0.8 M.

The features disclosed as being part of the second aspect of the invention can be in the second aspect of the invention, either alone or in combination.

The third aspect of the invention is drawn to a zirconium oxide recharging flow path. In any embodiment, the zirconium oxide recharging flow path can include (i) a water source and a base source; (ii) a zirconium oxide module inlet and a zirconium oxide module outlet; wherein the zirconium oxide module inlet and the zirconium oxide module outlet are fluidly connectable to a zirconium oxide sorbent module; (iii) at least one pump for flowing fluid from the water source and the base source to the zirconium oxide module inlet; and (iv) a zirconium oxide effluent line in fluid connection to the zirconium oxide module outlet.

In any embodiment, the base source can contain a mixture of sodium hydroxide and bleach.

In any embodiment, the base source can contain a concentrated solution of sodium hydroxide and bleach, and the zirconium oxide recharging flow path can include a static mixer, the static mixer fluidly connected to the water source and the base source.

In any embodiment, the base source can contain sodium hydroxide, and the zirconium oxide recharging flow path can have a heater.

The features disclosed as being part of the third aspect of the invention can be in the third aspect of the invention, either alone or in combination.

### BRIEF DESCRIPTION OF THE DRAWINGS

FIG. 1 is a flow chart of a method for disinfecting, recharging, and conditioning zirconium oxide with a combined base and bleach solution.
FIG. 2 is a flow chart of a method for disinfecting, recharging, and conditioning zirconium oxide including pretreatment of the zirconium oxide.
FIG. 3 is a flow chart of a method for disinfecting, recharging, and conditioning zirconium oxide including a mixture of peracetic acid and acetic acid as the disinfectant.
FIG. 4 is a flow chart of a method for disinfecting, recharging and conditioning zirconium oxide using a heated base solution as the disinfectant.
FIG. 5 is a zirconium oxide recharging flow path for disinfecting, recharging, and conditioning zirconium oxide with a combined base and bleach solution.
FIG. 6 is a zirconium oxide recharging flow path for disinfecting, recharging, and conditioning zirconium oxide with peracetic acid as the disinfectant.

### DETAILED DESCRIPTION OF THE INVENTION

Unless defined otherwise, all technical and scientific terms used generally have the same meaning as commonly understood by one of ordinary skill in the art.

The articles "a" and "an" are used to refer to one or to over one (*i.e.,* to at least one) of the grammatical object of the article. For example, "an element" means one element or over one element.

The term "acetic acid solution" refers to any aqueous solution containing acetic acid or CH₃COOH.

The term "acetic acid source" refers to any source of a fluid containing acetic acid.

An "acid solution" as used can be a Lewis acid or a Bronsted-Lowry acid dissolved in water. A Lewis acid is capable of accepting a lone pair of electrons. A Bronsted-Lowry acid is capable of donating a hydrogen ion to another compound.

The term "base solution" refers to any aqueous solution containing hydroxide ions and a pH of greater than 7.0.

A "base source" is any source of a base solution.

The term "bleach solution" refers to any aqueous solution containing sodium hypochlorite.

The term "comprising" includes, but is not limited to, whatever follows the word "comprising." Use of the term indicates the listed elements are required or mandatory but that other elements are optional and may be present.

A "concentrated" solution refers to a solution having at least one solute in a higher concentration than intended for use.

The terms "conditioning" or "to condition" refer to processes designed to allow safe and effective use of a component in dialysis.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

A "dilute" solution refers to a concentrated solution after addition of a solvent to lower the solute concentration.

The term "disinfectant solution" refers to any solution capable of destroying or removing bacterial contaminants from a reusable sorbent module.

The term "disinfectant source" refers to any source of a disinfectant solution.

The terms "flowing" or to "flow" "refer to the movement of a fluid, gas, or mixtures thereof.

The term "flow rate" refers to the volume of a fluid moved in a flow path in a given period of time.

The term "fluidly connectable," "fluidly connect," "for fluid connection," and the like, refer to the ability of providing for the passage of fluid, gas, or a combination thereof, from one point to another point. The two points can be within or between any one or more of compartments, modules, systems, components, and rechargers, all of any type. The connection can optionally be disconnected and then reconnected.

The terms "generating" or to "generate" refer to creating a fluid with a specified concentration, pH, temperature, and/or volume from one or more fluid sources.

A "heater" is a component capable of raising the temperature of a substance, container, or fluid.

The term "mixture" refers to a fluid having at least two components, the two components combined to form a substantially homogeneous substance.

The term "peracetic acid solution" refers to any aqueous solution containing peracetic acid or CH₃COOOH. The peracetic acid solution can also include hydrogen peroxide and acetic acid in equilibrium with the peracetic acid.

The term "pH" refers to the negative log of the H⁺ concentration in a fluid when stated in moles of H⁺ per liter of fluid volume.

The term "pump" refers to any device that causes the movement of fluids, gases, or combinations thereof, by applying suction or pressure.

"Recharging" refers to treating a sorbent material to restore the functional capacity of the sorbent material to put the sorbent material back into a condition for reuse or use in a new dialysis session. In some instances, the total mass, weight and/or amount of "rechargeable" sorbent materials remain the same. In some instances, the total mass, weight and/or amount of "rechargeable" sorbent materials change. Without being limited to any one theory of invention, the recharging process may involve exchanging ions bound to the sorbent material with different ions, which in some instances may increase or decrease the total mass of the system. However, the total amount of the sorbent material will in some instances be unchanged by the recharging process. Upon a sorbent material undergoing "recharging," the sorbent material can then be said to be "recharged."

The term "rinsing" or to "rinse" refers to flowing water through a component to remove substances from the component.

The term "sodium bicarbonate solution" refers to any aqueous solution containing sodium bicarbonate or NaHCO₃.

A "sorbent cartridge module" or "sorbent module" means a discreet component of a sorbent cartridge. Multiple sorbent cartridge modules can be fitted together to form a sorbent cartridge of two, three, or more sorbent cartridge modules. In some embodiments, a single sorbent cartridge module can contain all of the necessary materials for dialysis. In such cases, the sorbent cartridge module can be a "sorbent cartridge."

A "static mixer" is a component configured to receive fluids from one or multiple sources and to mix the fluids together. The static mixer may include components that agitate the fluids to further mixing.

The term "void volume" refers to a volume contained in a cartridge, not including a sorbent or any other material, through which liquid can flow. The term "void volume" can also refer to the volume of a cartridge or container available for a liquid or gas to fill the cartridge or container.

A "water source" is a fluid source from which water can be obtained.

"Zirconium oxide," also known as hydrous zirconium oxide, is a sorbent material that removes anions from a fluid, exchanging the removed anions for different anions.

A "zirconium oxide effluent line" is a fluid passageway, tube, or path of any kind into which fluid exiting a zirconium oxide sorbent module will flow.

A "zirconium oxide module inlet" is a connector through which a fluid, slurry, or aqueous solution can enter a zirconium oxide sorbent module.

A "zirconium oxide module outlet" is a connector through which a fluid, slurry, or aqueous solution can exit a zirconium oxide sorbent module.

A "zirconium oxide recharging flow path" is a path through which fluid can travel while recharging zirconium oxide in a reusable zirconium oxide sorbent module.

A "zirconium oxide sorbent module" is a sorbent module containing zirconium oxide.

A "zirconium phosphate module" or "zirconium phosphate sorbent module" is a sorbent module containing zirconium phosphate.

### Zirconium Oxide Recharging, Disinfection, and Conditioning

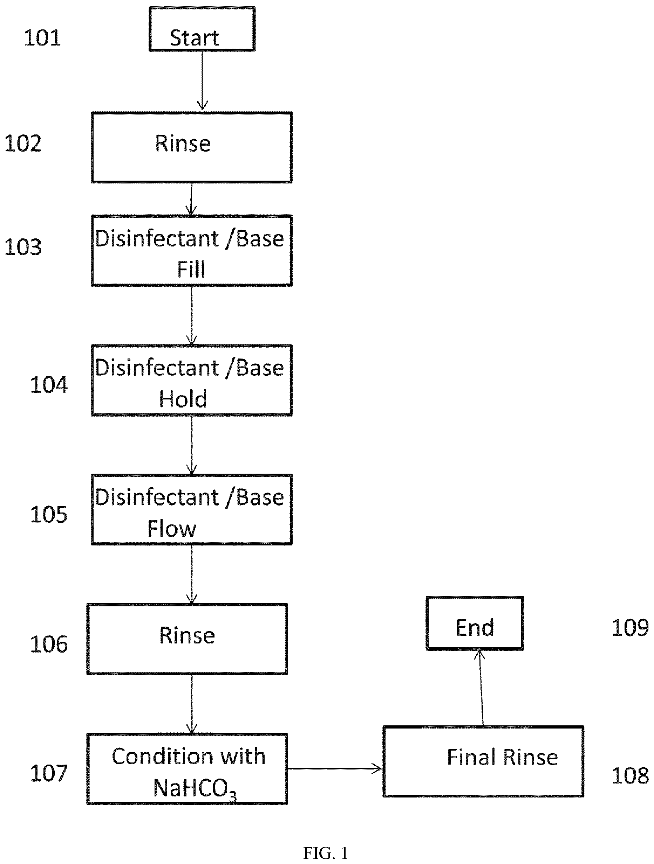

FIG. 1 is a flow chart illustrating a method for disinfecting, recharging, and conditioning zirconium oxide. To reuse zirconium oxide in dialysis, the method must achieve three steps. The cartridge must be disinfected to reduce the microbial load to acceptable levels, illustrated as steps **102-105;** the capacity of the zirconium oxide must be recovered, also occurring during steps **102-105;** and the zirconium oxide must be returned to an appropriate chemical state, or conditioned, which occurs during step **107.**

The method of disinfecting, recharging, and conditioning the zirconium oxide in a zirconium oxide sorbent module can begin in step **101.** During use in dialysis, phosphate and other anions are adsorbed by the zirconium oxide. In step **102,** the zirconium oxide sorbent module can optionally be rinsed with water to remove human dialysate waste. In some cases, the removal of human dialysate waste can make the disinfection of the zirconium oxide sorbent module more efficacious. The pre-rinsing in step **102** can include flowing water through the zirconium oxide sorbent module. The volume of water used can be any volume, including between 50 and 500% of the void volume of the zirconium oxide sorbent cartridge. However, the pre-rinsing step **102** is optional and can be omitted. In step **103,** the zirconium oxide sorbent module is filled with a mixture of disinfectant solution and a base. In a preferred embodiment, the disinfectant solution is bleach, or a sodium hypochlorite solution. The base can be any hydroxide base, including sodium hydroxide, lithium hydroxide, potassium hydroxide, or any other hydroxide base. In a preferred embodiment, the base is sodium hydroxide. The sodium hydroxide concentration can range from 0.2M to 2.0M, with a preferred concentration of about 0.8M. The bleach concentration can range from 0.01wt% to 2wt% with a preferred concentration of 0.10wt%. The preferred combined solution composition is 0.8M NaOH/0.10wt% bleach. By combining the disinfectant and base in a single solution, the recharge and disinfection of the zirconium oxide can occur at the same time. The hydroxide base recovers the capacity of the zirconium oxide by exchanging the phosphate and other anions for hydroxide anions while the bleach disinfects the zirconium oxide. Another benefit of combining the base and bleach is the increased stability of sodium hypochlorite at high pH.

The volume of base and bleach flowed through the zirconium oxide sorbent module can vary depending on the size of the zirconium oxide sorbent module. The volume can be between 100 and 200% or more of the void volume of the zirconium oxide sorbent module, ensuring complete filling with the base and bleach solution. In a preferred embodiment, the volume of base and bleach flowed through the zirconium oxide sorbent module in step **103** is about 150% of the void volume of the zirconium oxide sorbent module. For example, with a 0.3 L zirconium oxide sorbent module, 0.45 L of the base and bleach solution can be flowed through the module in step **103.** Any flow rate can be used in step **103,** including between 5 and 500 mL/min. In a preferred embodiment, the flow rate is about 55 mL/min.

In step **104,** the mixture of bleach and base solution is optionally held in the zirconium oxide sorbent module. Holding the bleach and base solution in the zirconium oxide sorbent module allows in-line neutralization of the effluent by aligning the zirconium oxide recharging process, which produces a basic effluent, with a zirconium phosphate recharging process, which produces an acidic effluent. The hold time can vary from 0 to 60 minutes depending on the hold time and flow rates used in the zirconium phosphate recharging process. In a preferred embodiment, the hold time is 25 minutes, which allows alignment with the zirconium phosphate recharging process. If the zirconium oxide is being recharged independently of zirconium phosphate, step **104** may be omitted.

In step **105,** the mixture of bleach and base solution are again flowed through the zirconium oxide sorbent module. The concentrations and flow rates of bleach and base used in step **105** can be the same as described with reference to step **103.** The total amount of bleach and base solution needed to recharge and disinfect the zirconium oxide can vary depending on the size of the zirconium oxide sorbent module, the concentrations of bleach and base in the solution, and the amount of phosphate or other anions adsorbed by the zirconium oxide during therapy. The total volume of bleach and base flowed through the zirconium oxide sorbent module can be between 1 and 3 L combined in steps **103** and **105.** In a preferred embodiment, the total volume of bleach and base flowed through the zirconium oxide sorbent module is about 1.9 L of a 0.8M NaOH/0.10wt% bleach solution, for a 310g zirconium oxide sorbent module with a capacity to remove 250-mmol of phosphate.

In step **106,** the zirconium oxide sorbent module is rinsed with water to remove the bleach and base solution. Removal of the bleach and base is necessary prior to reuse of the zirconium oxide sorbent module or for storage of the zirconium oxide sorbent module. The volume of rinse water flowed through the zirconium oxide sorbent module in step **106** can vary depending on the size of the zirconium oxide sorbent module. The volume can vary from 100% to 1000% of the zirconium oxide sorbent module void volume. In a preferred embodiment, the volume of rinse water flowed through the zirconium oxide sorbent module is 667% of the module void volume, or 2L for a zirconium oxide sorbent module with a 0.3L void volume. The rinse volume is chosen to reduce the residual bleach levels to 0.5-ppm total chlorine or less.

In step **107,** the zirconium oxide sorbent module is conditioned with a sodium bicarbonate solution. Conditioning of the zirconium oxide is necessary to place the zirconium oxide in a state usable in dialysis. Without conditioning, the zirconium oxide will consume total carbonate from the dialysate. Conditioning of the zirconium oxide with sodium bicarbonate places the zirconium oxide in pH equilibrium with the bicarbonate conditioning solution. The conditioning solution is preferably a sodium bicarbonate solution at the same pH as effluent from the zirconium phosphate sorbent module during therapy, and can be between 5.0 and 9.0 depending on the needs of the patient and system. In a preferred embodiment, the pH of the conditioning solution is about 6.5. By equilibrating the zirconium oxide during step **107** with the zirconium phosphate effluent pH, the zirconium oxide sorbent module can be used without adversely affecting the dialysate pH during therapy.

The conditioning step **107** can take place in a recharger or in a dialysis console prior to therapy. To ensure that the conditioning solution is at the proper pH, the conditioning solution, containing bicarbonate, can be flowed through a zirconium phosphate module that has already been recharged to the proper pH. The effluent leaving the zirconium phosphate module will be at the desired conditioning pH, and the zirconium oxide sorbent module will eventually equilibrate to the desired pH during conditioning. Alternatively, a separate conditioning solution can be flowed through the zirconium oxide sorbent module, with the separate conditioning solution premade to a desired pH. The separate conditioning solution can be prepared by any method known in the art. In one non-limiting example, the separate conditioning solution can be a pressured solution of sodium bicarbonate and carbon dioxide. Alternatively, a solution of sodium bicarbonate can be mixed with an acid, such as acetic acid or HCl to generate a conditioning solution having a desired pH. The pH of the conditioning solution can be any pH usable as a dialysate pH during therapy. In a preferred embodiment, the pH of the conditioning solution is about 6.5.

An optional final rinse step **108** can be included after conditioning. Storage of the zirconium oxide sorbent module with bicarbonate may increase the risk of microbial growth. The conditioning step **107** can be performed either in the recharger, or alternatively in the dialysis console just prior to use. If the conditioning step **107** is performed on the recharger, the zirconium oxide sorbent module can be rinsed in final rinse step **108** after conditioning to remove the sodium bicarbonate prior to storage. If the conditioning step **107** is performed in the dialysis console, or if the recharged zirconium oxide sorbent module will be immediately reused without storage, the final rinse step **108** can be omitted. In step **109,** the method can end and the zirconium oxide sorbent module can be used again in dialysis.

Although the method depicted in FIG. 1 uses a combined disinfectant and base solution, the disinfection and recharging of the zirconium oxide can be carried out with separate solutions. For example, a bleach solution can be flowed through the zirconium oxide sorbent module first, followed by a hydroxide base solution.

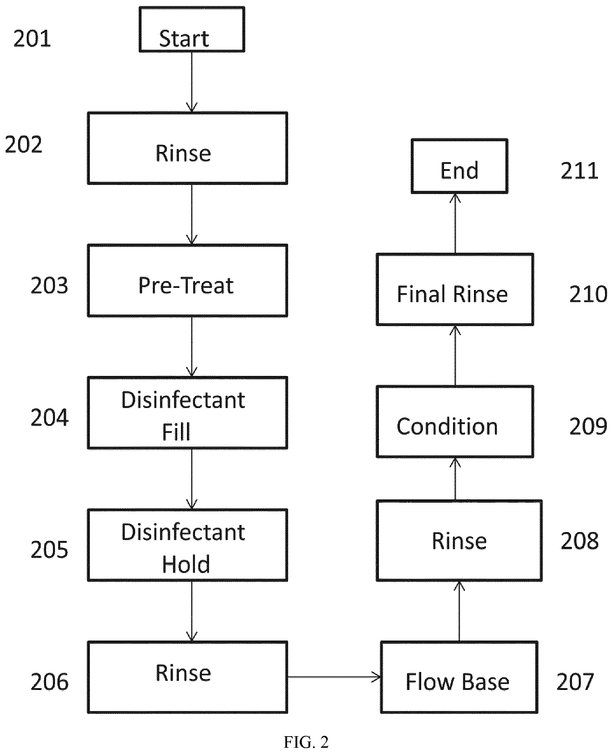

FIG. 2 illustrates a method for disinfecting, recharging, and conditioning zirconium oxide with a peracetic acid disinfectant solution. The peracetic acid disinfection solution can include hydrogen peroxide and acetic acid in equilibrium with the peracetic acid. The method can start in step **201.** In step **202,** the zirconium oxide sorbent module can optionally be rinsed with water to remove human dialysate waste from the zirconium oxide sorbent module. In step **203,** the zirconium oxide sorbent module can be pre-treated with an acid solution, optionally containing a sodium acetate/acetic acid buffer. Without pretreatment, the zirconium oxide consumes peracetic acid, necessitating up to four times as much peracetic acid for disinfection as is needed with pretreatment. The zirconium oxide, prior to pretreatment, may contain bicarbonate, which can react with the peracetic acid during disinfection. The pretreatment with an acid solution may remove the bicarbonate and convert the bicarbonate to acetate, allowing disinfection with less peracetic acid. The pretreatment solution can be any acid solution capable of removing a substantial amount of bicarbonate from the zirconium oxide, including a buffer containing between 0.3M and 1.1M sodium acetate and between 0.2M and 0.8M acetic acid. The pretreatment solution can optionally contain sodium chloride as well. The sodium chloride concentration in the pretreatment solution can be between 2.5 M and 4.9 M. Advantageously, a zirconium phosphate sorbent module can be recharged with an acetic acid/sodium acetate buffer and sodium chloride, allowing recharging of a zirconium phosphate module and pretreatment of a zirconium oxide sorbent module with the same solution.

In step **204,** the zirconium oxide sorbent module can be filled with a peracetic acid disinfectant. The peracetic acid solution can have any concentration of peracetic acid sufficient to disinfect the zirconium oxide sorbent module, including between 0.5 and 2 wt%. In a preferred embodiment, the peracetic acid solution is 1 wt% in water. The volume of peracetic acid flowed into the zirconium oxide sorbent module should be enough to completely fill the zirconium oxide sorbent module, and can be between 100% to 200% or more of the void volume of the zirconium oxide sorbent module. In a preferred embodiment, a volume of peracetic acid solution flowed into the zirconium oxide sorbent module is 150% of the module void volume.

In step **205,** the peracetic acid disinfectant solution can be held in the zirconium oxide sorbent module for a sufficient length of time to ensure complete disinfection of the zirconium oxide sorbent module. The hold time can be any length of time, including between 5 and 30 minutes, between 5 and 10 minutes, between 5 and 15 minutes, between 10 and 20 minutes, between 15 and 30 minutes or between 20 and 30 minutes. The length of the hold time can depend on the temperature of the zirconium oxide sorbent module, and in a preferred embodiment the hold time can be 15 minutes at 22 °C. In step **206,** the zirconium oxide sorbent module can be rinsed with water, allowing a buffer of water to be present between the peracetic acid solution and the subsequent base solution.

In step **207** a hydroxide base solution can be flowed through the zirconium oxide sorbent module. The hydroxide base solution can be the same solutions described with reference to FIG. 1, without the added bleach. The volume of base flowed through the zirconium oxide sorbent module can be between 1 and 3 L. In a preferred embodiment, the total volume of base flowed through the zirconium oxide sorbent module is about 1.9 L of a 0.8M NaOH solution, for a 310g ZO cartridge with a capacity to remove 250-mmol of phosphate. Any flow rate can be used in step **207,** including between 5 and 500 mL/min. In a preferred embodiment, the flow rate is about 55 mL/min.

An optional rinse step **208** can be included to remove base from the cartridge in cases where conditioning step **209** is not performed on the recharger and the cartridge is stored until the next therapy.

In step **209,** the zirconium oxide sorbent module can be conditioned with a sodium bicarbonate solution. The conditioning process of step **209** can use the same solution, volume, and flow rates as the conditioning process described with respect to FIG. 1. An optional final rinse step **210** can be included to remove the bicarbonate solution from the zirconium oxide sorbent module for storage. In step **211,** the method can end with a zirconium oxide sorbent module usable in dialysis.

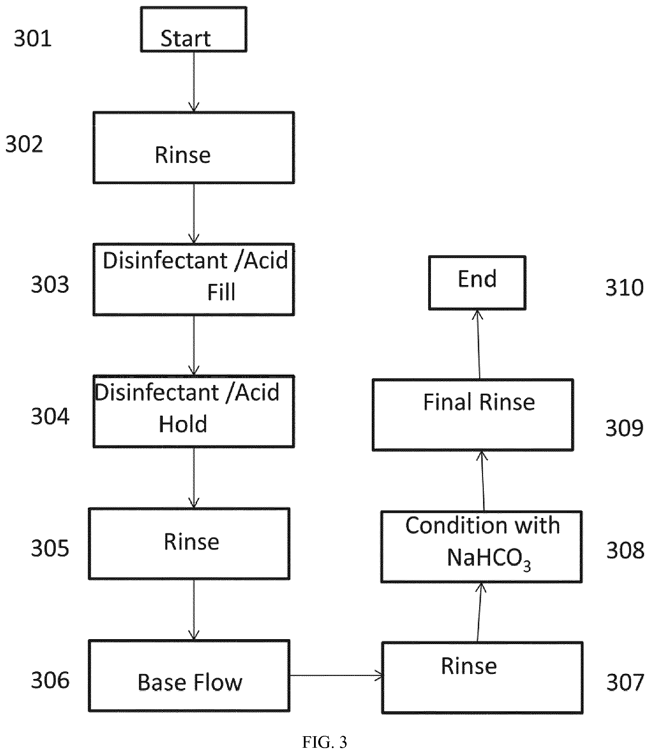

FIG. 3 illustrates an alternative method of disinfecting, recharging, and conditioning zirconium oxide in a zirconium oxide sorbent module. The method can start in step **301,** with a used zirconium oxide sorbent module. In step **302,** the zirconium oxide sorbent module can optionally be pre-rinsed with water to remove human dialysate waste. Instead of pretreating the zirconium oxide sorbent module, in step **303,** the zirconium oxide sorbent module can be filled with a mixture of a peracetic acid solution and an acetic acid solution. The acetic acid may stabilize the peracetic acid, keeping the peracetic acid/acetic acid equilibrium shifted towards peracetic acid formation. The mixture used in step **303** can have any concentration of peracetic acid and acetic acid capable of disinfecting the zirconium oxide while keeping the peracetic acid equilibrium shifted towards the peracetic side. The concentration of peracetic acid in the solution can be between 1.0 and 3.0%, and the concentration of acetic acid can be between 5.0 and 15.0%. In a preferred embodiment, the disinfectant and acid solution is about 2% peracetic acid and about 10% acetic acid.

In step **304,** the mixture of peracetic acid and acetic acid disinfectant solution can be held in the zirconium oxide sorbent module for a sufficient length of time to ensure complete disinfection of the zirconium oxide sorbent module. The hold time can be any length of time, including between 5 and 30 minutes, between 5 and 10 minutes, between 5 and 15 minutes, between 10 and 20 minutes, between 15 and 30 minutes or between 20 and 30 minutes. The length of the hold time can depend on the temperature of the zirconium oxide sorbent module, and in a preferred embodiment the hold time can be 15 minutes at 22 °C. In step **305,** the zirconium oxide sorbent module can be rinsed with water, allowing a buffer of water to be present between the peracetic acid solution and the subsequent base solution.

In step **306,** a hydroxide base solution can be flowed through the zirconium oxide sorbent module. The hydroxide base solution can be the same solutions described with reference to FIG. 1, without the added bleach. The volume of base flowed through the zirconium oxide sorbent module can be between 1 and 3 L. In a preferred embodiment, the total volume of base flowed through the zirconium oxide sorbent module is about 1.9 L of a 0.8M NaOH solution, for a 310g ZO cartridge with a capacity to remove 250-mmol of phosphate. Any flow rate can be used in step **306,** including between 5 and 500 mL/min. In a preferred embodiment, the flow rate is about 55 mL/min.

An optional rinse step **307** can be included to remove base from the cartridge in cases where conditioning step **308** is not performed on the recharger and the cartridge is stored until the next therapy.

In step **308,** the zirconium oxide sorbent module can be conditioned with a sodium bicarbonate solution. The conditioning process of step **308** can use the same solution, volume, and flow rates as the conditioning process described with respect to FIG. 1. An optional final rinse step **309** can be included to remove the bicarbonate from the zirconium oxide sorbent module for storage. In step **310,** the method can end with a zirconium oxide sorbent module usable in dialysis.

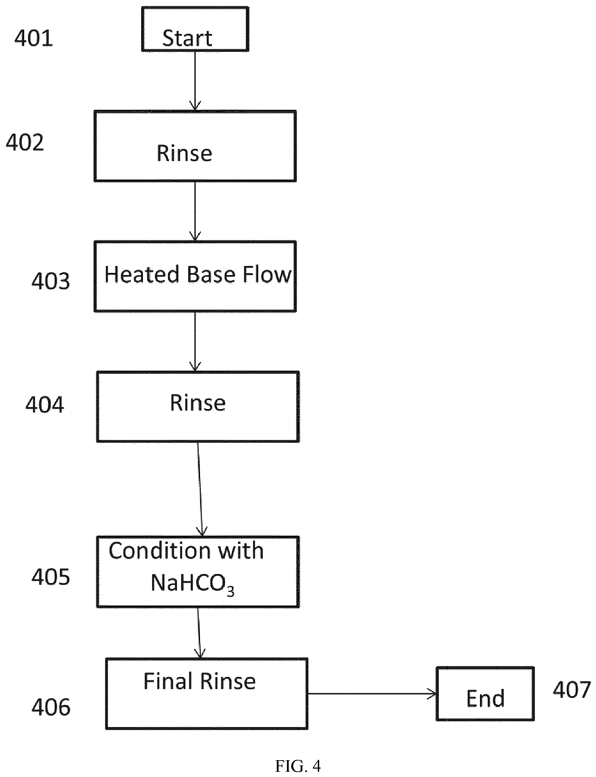

FIG. 4 illustrates an alternative method of disinfecting, recharging, and conditioning zirconium oxide in a zirconium oxide sorbent module using heated base as the disinfectant. The method can start in step **401,** with a used zirconium oxide sorbent module. In step **402,** the zirconium oxide sorbent module can optionally be pre-rinsed with water to remove human dialysate waste. In step **403,** a heated base solution can be flowed through the zirconium oxide sorbent module. The heated base solution can be any hydroxide base capable of disinfecting and recovering the capacity of the zirconium oxide sorbent module at elevated temperatures. In a preferred embodiment, the base is sodium hydroxide. The sodium hydroxide concentration can range from 0.2M to 2.0M, with a preferred concentration of about 0.8M. The base solution can be heated to any temperature capable of disinfecting the zirconium oxide sorbent module, including between 40 and 100°C. In a preferred embodiment, the base solution can be heated to about 60°C. Any flow rate can be used in step **403,** including between 5 and 500 mL/min. In a preferred embodiment, the flow rate is about 55 mL/min. The volume of heated base flowed through the zirconium oxide sorbent module can be between 1 and 3 L. In a preferred embodiment, the total volume of heated base flowed through the zirconium oxide sorbent module is about 1.9 L of a 0.8M NaOH solution, for a 310g ZO cartridge with a capacity to remove 250-mmol of phosphate. Using heated base as a disinfectant allows disinfection and recharging of the zirconium oxide sorbent module in a single step. An optional rinse step **404** can be included to remove base from the cartridge in cases where conditioning step **405** is not performed on the recharger and the cartridge is stored until the next therapy.

In step **405,** the zirconium oxide sorbent module can be conditioned with a sodium bicarbonate solution. The conditioning process of step **405** can use the same solution, volume, and flow rates as the conditioning process described with respect to FIG. 1. An optional final rinse step **406** can be included to remove the bicarbonate from the zirconium oxide sorbent module for storage. In step **407,** the method can end with a zirconium oxide sorbent module usable in dialysis.

The direction of flow through the zirconium oxide sorbent module during the methods of FIG.'s 1-4 can optionally be in an opposite direction as dialysate flows through the zirconium oxide sorbent module during therapy. During therapy, spent dialysate enters the bottom of the module, flows upwards against gravity, and exits the top of the cartridge. During disinfection, recharging, and conditioning, the solutions can enter the zirconium oxide sorbent module at the top and exit through the bottom, flowing with gravity. Alternatively, the spent dialysate can travel downward through the zirconium oxide sorbent module during therapy, and the solutions can flow upwardly through the zirconium oxide sorbent module during disinfection, recharging, and conditioning. Alternatively, the direction of flow of the solutions during recharging can be the same as the flow direction during therapy.

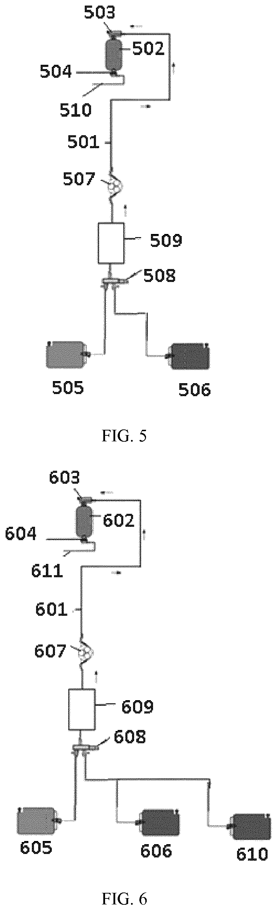

FIG. 5 is a non-limiting example of a zirconium oxide recharging flow path **501** that can recharge the zirconium oxide as illustrated in FIG. 1. A zirconium oxide sorbent module **502** can connect to the zirconium oxide recharging flow path **501** through zirconium oxide sorbent module inlet **503** and zirconium oxide outlet **504.** A zirconium oxide effluent line **510** can be fluidly connected to the zirconium oxide outlet **504** for removal of solutions after the solutions pass through the zirconium oxide sorbent module **502.** Pump **507** provides a driving force for flowing fluids through the zirconium oxide recharging flow path **501.** A combined disinfectant solution and base solution source **505,** containing bleach and a hydroxide base, such as sodium hydroxide, can be fluidly connected to the zirconium oxide recharging flow path **501.** Water source **506** is also fluidly connected to the zirconium oxide recharging flow path **501.** Valve **508** controls the movement of fluid through the zirconium oxide recharging flow path **501** and zirconium oxide sorbent module **502.** A static mixer **509** can optionally be included to ensure complete mixing of solutions prior to entering the zirconium oxide sorbent module **502** and can be used to generate a dilute solution from the combined disinfectant solution and base solution source **505** when a concentrated bleach and base solution is used. One of skill in the art will understand that different pump and valve arrangements can be used with the system illustrated in FIG. 5. For example, the combined disinfectant solution and base solution source **505** and water source **506** can be connected to the zirconium oxide recharging flow path **501** through separate pumps, allowing simultaneous addition of water, base, and disinfectant to the zirconium oxide recharging flow path **501.** Further, separate base and bleach solution sources can be used and simultaneously flowed to the static mixer **509.** The bleach and base solution can be mixed in the static mixer **509** prior to flowing through the zirconium oxide sorbent module **502.** A heater (not shown) can optionally be included in the zirconium oxide recharging flow path **501** for heating the solutions in cases where the water in the water source **506** is below room temperature, or to increase recharging and disinfection efficiency by using temperatures greater than room temperature.

FIG. 6 illustrates a non-limiting embodiment of a zirconium oxide recharging flow path 601 that can recharge the zirconium oxide as illustrated in FIG. 2. A zirconium oxide sorbent module **602** can connect to the zirconium oxide recharging flow path **601** through zirconium oxide sorbent module inlet **603** and zirconium oxide outlet **604.** A zirconium oxide effluent line **611** can be fluidly connected to the zirconium oxide outlet **604** for removal of solutions after the solutions pass through the zirconium oxide sorbent module **602.** Pump **607** provides a driving force for flowing fluids through the zirconium oxide recharging flow path **601.** A disinfectant and acetic acid source **605,** containing acetic acid and peracetic acid is fluidly connected to the zirconium oxide recharging flow path **601.** Base source **606** can contain a hydroxide base, such as sodium hydroxide. Water source **610** is also fluidly connected to the zirconium oxide recharging flow path **601.** The water source **610** can contain any type of water, including deionized water. Valve **608** controls the movement of fluid through the zirconium oxide recharging flow path **601** and zirconium oxide sorbent module **602.** A static mixer **609** can optionally be included to ensure complete mixing of solutions prior to entering the zirconium oxide sorbent module **602.** The static mixer **609** allows the solutions from disinfectant and acetic acid source **605** or base source **606** to be concentrated solutions, which can be diluted inline with water from the water source **610** in the static mixer **609.** One of skill in the art will understand that different pump and valve arrangements can be used with the system illustrated in FIG. 6. For example, the disinfectant and acetic acid source **605,** base source **606,** and water source **610** can be connected to the zirconium oxide recharging flow path **601** through separate pumps, allowing simultaneous addition of water, base, and/or disinfectant to the zirconium oxide recharging flow path **601.**

The solutions in the disinfectant and acetic acid source **605** and base source **606** can be any concentration, including the concentrations described with reference to FIG. 3. Alternatively, the solutions in disinfectant and acetic acid source **605** and/or base source **606** can be concentrated solutions. The base solution can have a sodium hydroxide concentration higher than that to be used in recharging the zirconium oxide sorbent module **602.** The base solution and water can be simultaneously flowed to static mixer **609** to dilute the concentrated base solution in the recharging steps generating a dilute base solution usable for recharging the zirconium oxide. Similarly, a concentrated disinfectant and acetic acid solution and water can be simultaneously flowed to static mixer **609** to dilute the disinfectant and acetic acid solution during the disinfecting steps, generating a dilute disinfectant and acetic acid solution for use in disinfecting the zirconium oxide. Alternatively, the disinfectant source can contain only peracetic acid, and the combined disinfectant acetic acid solution can be generated inline by flowing both the peracetic acid solution and an acetic acid solution from a separate acid source (not shown) to the static mixer **609.** Similarly, a separate brine source containing acetic acid, sodium acetate, and optionally sodium chloride can be included for pretreating the zirconium oxide prior to flowing solely peracetic acid through the zirconium oxide sorbent module **602** for disinfection. Advantageously, the pretreatment solution can be used as a recharging solution for recharging zirconium phosphate in a zirconium phosphate recharging flow path (not shown), allowing the same disinfectant and brine solution to be used for both zirconium oxide and zirconium phosphate. A heater (not shown) can optionally be included in the zirconium oxide recharging flow path **601** for heating the solutions in cases where the water in the water source **610** is below room temperature, or to increase recharging and disinfection efficiency by using temperatures greater than room temperature.

One skilled in the art will understand that various combinations and/or modifications and variations can be made in the described systems and methods depending upon the specific needs for operation. Moreover features illustrated or described as being part of an aspect of the invention may be used in the aspect of the invention, either alone or in combination.

The invention may be described by reference to the following numbered paragraphs:-
1. A method of recharging zirconium oxide in a zirconium oxide sorbent module, comprising the steps of:
   flowing a disinfectant solution through the zirconium oxide sorbent module and flowing a base solution through the zirconium oxide sorbent module; wherein the step of flowing the disinfectant solution through the zirconium oxide sorbent module comprises any one of:
   a) flowing an acid solution and a peracetic acid solution through the zirconium oxide sorbent module;
   b) flowing a heated base solution through the zirconium oxide sorbent module; or
   c) flowing a bleach solution through the zirconium oxide sorbent module.
2. The method of paragraph 1; wherein the base solution is a sodium hydroxide solution.
3. The method of paragraph 1; wherein the step of flowing the acid solution and the peracetic acid solution through the zirconium oxide sorbent module comprises flowing a mixture of acetic acid and peracetic acid through the zirconium oxide sorbent module.
4. The method of paragraph 3; wherein the mixture of acetic acid and peracetic acid comprises between 1% and 3% peracetic acid and between 5% and 15% acetic acid.
5. The method of paragraph 1; wherein the step of flowing the acid solution and the peracetic acid solution through the zirconium oxide sorbent module comprises first flowing the acid solution through the zirconium oxide sorbent module; and then flowing the peracetic acid solution through the zirconium oxide sorbent module.
6. The method of paragraph 5, wherein the acid solution contains acetic acid and sodium acetate.
7. The method of paragraph 6, wherein the acid solution contains between 0.3M and 1.1M sodium acetate and between 0.2M and 0.8M acetic acid.
8. The method of paragraph 7, wherein the acid solution further contains between 2.5 M and 4.9 M sodium chloride.
9. The method of paragraph 1, wherein the steps of flowing the base solution and flowing the disinfectant solution through the zirconium oxide sorbent module comprise flowing a mixture of the base solution and disinfectant solution through the zirconium oxide sorbent module.
10. The method of paragraph 9, wherein the mixture of the base solution and disinfectant solution is a mixture of sodium hydroxide and bleach.
11. The method of paragraph 10, wherein the mixture of sodium hydroxide and bleach contains between 0.01% and 2.0 % bleach and between 0.2 M and 2.0 M sodium hydroxide.
12. The method of paragraph 10, wherein a flow rate of the mixture of the base solution and disinfectant solution is between 5 and 500 mL/min.
13. The method of paragraph 10, wherein a volume of the mixture of the base solution and disinfectant solution flowed through the zirconium oxide sorbent module is between 1.0 and 3.0 L.
14. The method of paragraph 1, further comprising the step of rinsing the zirconium oxide sorbent module with water after flowing the base solution through the zirconium oxide sorbent module.
15. The method of paragraph 14, wherein a volume of water flowed through the zirconium oxide sorbent module to rinse the zirconium oxide sorbent module is between 100% and 1000% of a void volume of the zirconium oxide sorbent module.
16. The method of paragraph 1, further comprising the step of conditioning the zirconium oxide sorbent module by flowing a sodium bicarbonate solution through the zirconium oxide sorbent module.
17. The method of paragraph 16, wherein a pH of the sodium bicarbonate solution is between 5.0 and 9.0.
18. The method of paragraph 16, further comprising the step of flowing the sodium bicarbonate solution through a zirconium phosphate sorbent module prior to flowing the sodium bicarbonate solution through the zirconium oxide sorbent module.
19. The method of paragraph 1, further comprising the step of generating any one of the disinfectant solution and base solution in a zirconium oxide recharging flow path.
20. The method of paragraph 1, wherein any one:
   a) the step of flowing the disinfectant solution through the zirconium oxide sorbent module comprises flowing a concentrated disinfectant solution and water to a static mixer to generate a dilute disinfectant solution; and flowing the dilute disinfectant solution through the zirconium oxide sorbent module;
   b) the step of flowing the base solution through the zirconium oxide sorbent module comprises flowing a concentrated base solution and water to a static mixer to generate a dilute base solution; and flowing the dilute base solution through the zirconium oxide sorbent module; or
   c) combinations thereof.
21. A zirconium oxide recharging flow path, comprising:
   i) a water source, a disinfectant source, a base source, and an acetic acid source;
   ii) a zirconium oxide module inlet and a zirconium oxide module outlet; wherein the zirconium oxide module inlet and the zirconium oxide module outlet are fluidly connectable to a zirconium oxide sorbent module;
   iii) at least one pump for flowing fluid from the water source, the disinfectant source, the base source, and the acetic acid source to the zirconium oxide module inlet; and
   iv) a zirconium oxide effluent line fluidly connectable to the zirconium oxide module outlet.
22. The zirconium oxide recharging flow path of paragraph 21, wherein the disinfectant source is a peracetic acid source.
23. The zirconium oxide recharging flow path of paragraph 21, wherein the acetic acid source contains a mixture of acetic acid, sodium chloride, and sodium acetate.
24. The zirconium oxide recharging flow path of paragraph 21, wherein the disinfectant source is a peracetic acid solution having a concentration in a range between 0.5 % and 2% of peracetic acid in water.
25. The zirconium oxide recharging flow path of paragraph 23, wherein the acetic acid source contains sodium chloride between 2.5 M and 4.9 M, sodium acetate between 0.3 M and 1.1 M, and acetic acid between 0.2 M and 0.8 M.
26. A zirconium oxide recharging flow path, comprising:
   i) a water source and a base source;
   ii) a zirconium oxide module inlet and a zirconium oxide module outlet; wherein the zirconium oxide module inlet and the zirconium oxide module outlet are fluidly connectable to a zirconium oxide sorbent module;
   iii) at least one pump for flowing fluid from the water source and the base source to the zirconium oxide module inlet; and
   iv) a zirconium oxide effluent line in fluid connection to the zirconium oxide module outlet.
27. The zirconium oxide recharging flow path of paragraph 26, wherein the base source contains a mixture of sodium hydroxide and bleach.
28. The zirconium oxide recharging flow path of paragraph 27, wherein the base source contains a concentrated solution of sodium hydroxide and bleach, and the zirconium oxide recharging flow path further comprises a static mixer, the static mixer fluidly connected to the water source and the base source.
29. The zirconium oxide recharging flow path of paragraph 26, wherein the base source contains sodium hydroxide, and the zirconium oxide recharging flow path further comprises a heater.

## Claims

1. A method of recharging zirconium oxide in a zirconium oxide sorbent module,
comprising the steps of:
flowing a disinfectant solution through the zirconium oxide sorbent module and flowing a base solution through the zirconium oxide sorbent module; wherein the step of flowing the disinfectant solution through the zirconium oxide sorbent module comprises any one of:
a) flowing an acid solution and a peracetic acid solution through the zirconium oxide sorbent module;
b) flowing a heated base solution through the zirconium oxide sorbent module; or
c) flowing a bleach solution through the zirconium oxide sorbent module.

2. The method of claim 1; wherein the base solution is a sodium hydroxide solution.

3. The method of claim 1 or claim 2; wherein the step of flowing the acid solution and the peracetic acid solution through the zirconium oxide sorbent module comprises flowing a mixture of acetic acid and peracetic acid through the zirconium oxide sorbent module; preferably wherein the mixture of acetic acid and peracetic acid comprises between 1% and 3% peracetic acid and between 5% and 15% acetic acid.

4. The method of claim 1 or claim 2; wherein the step of flowing the acid solution and the peracetic acid solution through the zirconium oxide sorbent module comprises first flowing the acid solution through the zirconium oxide sorbent module; and then flowing the peracetic acid solution through the zirconium oxide sorbent module; preferably, wherein the acid solution contains acetic acid and sodium acetate; preferably, wherein the acid solution contains between 0.3M and 1. 1M sodium acetate and between 0.2M and 0.8M acetic acid; most preferably, wherein the acid solution further contains between 2.5 M and 4.9 M sodium chloride.

5. The method of any preceding claim, wherein the steps of flowing the base solution and flowing the disinfectant solution through the zirconium oxide sorbent module comprise flowing a mixture of the base solution and disinfectant solution through the zirconium oxide sorbent module.

6. The method of claim 5, wherein the mixture of the base solution and disinfectant solution is a mixture of sodium hydroxide and bleach; preferably, wherein the mixture of sodium hydroxide and bleach contains between 0.01% and 2.0 % bleach and between 0.2 M and 2.0 M sodium hydroxide; and/or wherein a flow rate of the mixture of the base solution and disinfectant solution is between 5 and 500 mL/min; and/or wherein a volume of the mixture of the base solution and disinfectant solution flowed through the zirconium oxide sorbent module is between 1.0 and 3.0 L.

7. The method of any preceding claim, further comprising the step of rinsing the zirconium oxide sorbent module with water after flowing the base solution through the zirconium oxide sorbent module; preferably, wherein a volume of water flowed through the zirconium oxide sorbent module to rinse the zirconium oxide sorbent module is between 100% and 1000% of a void volume of the zirconium oxide sorbent module.

8. The method of any preceding claim, further comprising the step of conditioning the zirconium oxide sorbent module by flowing a sodium bicarbonate solution through the zirconium oxide sorbent module; prefably, wherein a pH of the sodium bicarbonate solution is between 5.0 and 9.0; and/or further comprising the step of flowing the sodium bicarbonate solution through a zirconium phosphate sorbent module prior to flowing the sodium bicarbonate solution through the zirconium oxide sorbent module.

9. The method of any preceding claim, further comprising the step of generating any one of the disinfectant solution and base solution in a zirconium oxide recharging flow path.

10. The method of claim 1, wherein any one:
a) the step of flowing the disinfectant solution through the zirconium oxide sorbent module comprises flowing a concentrated disinfectant solution and water to a static mixer to generate a dilute disinfectant solution; and flowing the dilute disinfectant solution through the zirconium oxide sorbent module;
b) the step of flowing the base solution through the zirconium oxide sorbent module comprises flowing a concentrated base solution and water to a static mixer to generate a dilute base solution; and flowing the dilute base solution through the zirconium oxide sorbent module; or
c) combinations thereof.

11. A zirconium oxide recharging flow path, comprising:
i) a water source, a disinfectant source, a base source, and an acetic acid source;
ii) a zirconium oxide module inlet and a zirconium oxide module outlet; wherein the zirconium oxide module inlet and the zirconium oxide module outlet are fluidly connectable to a zirconium oxide sorbent module;
iii) at least one pump for flowing fluid from the water source, the disinfectant source, the base source, and the acetic acid source to the zirconium oxide module inlet; and
iv) a zirconium oxide effluent line fluidly connectable to the zirconium oxide module outlet.

12. The zirconium oxide recharging flow path of claim 11, wherein the disinfectant source is a peracetic acid source; and/or wherein the acetic acid source contains a mixture of acetic acid, sodium chloride, and sodium acetate; and/or wherein the disinfectant source is a peracetic acid solution having a concentration in a range between 0.5 % and 2% of peracetic acid in water.

13. The zirconium oxide recharging flow path of claim 12, wherein the acetic acid source contains sodium chloride between 2.5 M and 4.9 M, sodium acetate between 0.3 M and 1.1 M, and acetic acid between 0.2 M and 0.8 M.

14. A zirconium oxide recharging flow path, comprising:
i) a water source and a base source;
ii) a zirconium oxide module inlet and a zirconium oxide module outlet; wherein the zirconium oxide module inlet and the zirconium oxide module outlet are fluidly connectable to a zirconium oxide sorbent module;
iii) at least one pump for flowing fluid from the water source and the base source to the zirconium oxide module inlet; and
iv) a zirconium oxide effluent line in fluid connection to the zirconium oxide module outlet.

15. The zirconium oxide recharging flow path of claim 14, wherein the base source contains a mixture of sodium hydroxide and bleach; preferably, wherein the base source contains a concentrated solution of sodium hydroxide and bleach, and the zirconium oxide recharging flow path further comprises a static mixer, the static mixer fluidly connected to the water source and the base source; and/or wherein the base source contains sodium hydroxide, and the zirconium oxide recharging flow path further comprises a heater.
